(19) Europäisches Patentamt
European Patent Office
Office européen des brevets

(11) Publication number: **0 248 885 B1**

# (12) EUROPEAN PATENT SPECIFICATION

(45) Date of publication of patent specification: **05.08.92**

(51) Int. Cl.$^5$: **A61J 3/00**, A61L 9/04, A61K 31/335, A61K 31/12

(21) Application number: **87900445.5**

(22) Date of filing: **28.11.86**

(86) International application number: **PCT/US86/02583**

(87) International publication number: **WO 87/03473 (18.06.87 87/13)**

## (54) TRANSDERMAL DELIVERY OF DRUGS.

(30) Priority: **04.12.85 US 804661**
**21.08.86 US 899049**

(43) Date of publication of application: **16.12.87 Bulletin 87/51**

(45) Publication of the grant of the patent: **05.08.92 Bulletin 92/32**

(84) Designated Contracting States: **BE FR IT**

(56) References cited:
**GB-A- 2 142 237**
**US-A- 3 474 176**
**US-A- 3 921 636**
**US-A- 3 964 482**
**US-A- 3 996 934**

(73) Proprietor: **HSIEH, Dean**
**1400 Millstone River Road**
**Belle Mead, NJ 08502(US)**

(72) Inventor: **HSIEH, Dean**
**1400 Millstone River Road**
**Belle Mead, NJ 08502(US)**

(74) Representative: **Ellis-Jones, Patrick George Armine et al**
**J.A. KEMP & CO. 14 South Square Gray's Inn**
**London WC1R 5EU(GB)**

**EP 0 248 885 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the topical, nasal, vaginal and other routes of administration of physiologically active agents such as drugs to humans and animals. It particularly relates to systems for the delivery of drugs across body membranes and providing an enhanced rate of passage across such membranes.

### BACKGROUND OF THE INVENTION

Administration of drugs using transdermal delivery systems is well known and documented in both the patent and scientific literature.

Administration using transdermal drug delivery systems has certain advantages over the conventional methods of oral and systemic administration. These advantages include: (1) minimizing drug exposure by allowing a significant reduction in dosage; (2) providing long-term therapy in a single dose thereby increasing patient compliance; (3) avoiding the risks and inconveniences of intravenous or intramuscular therapy; (4) rendering possible the use of drugs with short biological half-lives; (5) allowing immediate termination of drug input by simply removing the material containing the drug; and (6) avoiding the possible inactivation of a drug when it first passes through the liver after oral administration.

Examples of drugs which have been administered transdermally include scopolamine, nitroglycerin, clonidine, estradiol, antibiotics (e.g., erythromycin, lincomycin and the like), antifungal agents, and sunsreens. Many of these drugs, e.g., clonidine, scopolamine, and nitrogylcerin are of such chemical structure that they can permeate the skin and other body membranes to provide sufficiently high theraputic doses for most purposes. However, when higher theraputic levels are required, or when the drug itself, e.g., estradiol diacetate, does not permeate or cannot sufficiently permeate the skin to provide the desired level of drug concentration, it becomes necessary to use adjuvants which enhance the rate of penetration of the drug. Generally, for transdermal formulation of most drug entities adjuvants are required.

Compounds which have been used as adjuvants include dimethyl sulfoxide and homologs thereof, 1-alkyl-azacycloheptan-2-ones (azone), N,N-dimethyl-m-toluidine, long chain aliphatic alkanes, alcohols, carboxylic acids and esters and substituted (e.g., halo) derivatives thereof, cyclohexylalkanols, phenylalkanols, mixtures of siloxanes with either amides or urea derivatives, $C_{3-4}$ diols and ethers and esters thereof, mixtures of $C_{3-4}$ diols with surfactants, eucalyptol, urea, a mixture of 2-pyrrolidone and dimethyl formamide, 1,3-dimethyl-2-imidazolidinone, dicyclohexylmethylamine oxide, mixture of hexane and ethylene glycol monomethyl ether, a mixture of ricinoleyl alcohol and an ethoxylated partial glycerine of a $C_{6-12}$ saturated fatty acid, N-substituted-diisopropylamines, and compounds of the formula

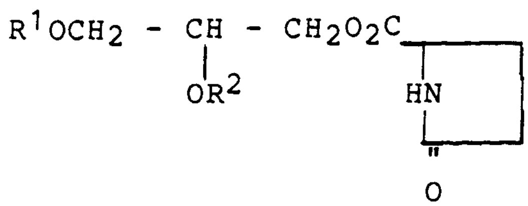

wherein $R^1$ and $R^2$ are hydrogen, $C_{1-25}$ alkyl, $C_{2-25}$ alkenyl, $C_{1-24}$ alkyl carbonyl, or $C_{2-24}$ alkenyl carbonyl.

While all of the above-listed adjuvants do serve to enhance the transdermal absorption of drugs, they possess certain drawbacks in that (i) some are regarded as toxic (e.g., dimethyl sulfoxide); (ii) some irritate the skin (e.g., surfactants); (iii) some on prolonged use have a thinning effect on the skin (e.g., oleic acid); and (iv) some change the intactness of the skin structure, resulting in a change in the diffusability of the drug (e.g., azone).

US-A-3964482 discloses the use of cycloaliphatic and aromatic ketones having 4 to 10 carbon atoms as transporting aids.

### DESCRIPTION OF THE INVENTION

It is an object of this invention to provide drug containing compositions which have an enhanced rate of passage across body membranes, the adjuvants being nontoxic, do not exert any physiological effects in the body other than enhancing the rate of passage of drugs across body membranes and have a minimal

effect on the structure of the skin after prolonged use.

In accordance with this invention it has been found that the addition to a composition containing an effective amount of a drug and a lactone or a cyclic ketone of the formula (I) or a cyclic anhydrides or ester of the formula (II)

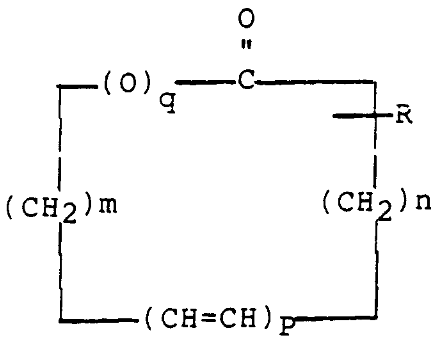

Formula I

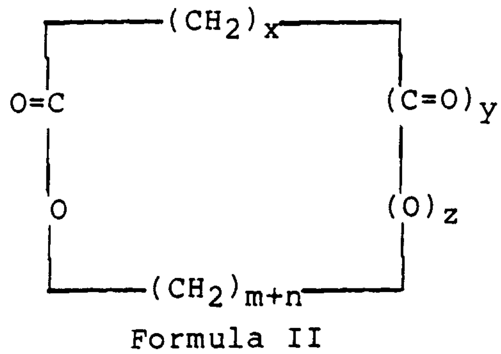

Formula II

wherein m and n are integers having a value from 1 to 20 with the proviso that m + n is at least 11 and not greater than 25, p is an integer having a value of 0 or 1, q is an integer having a value of 0 or 1, and R is hydrogen or an alkyl group containing from 1 to 6 carbon atoms, which may be straight chained or branched, will enhance the rate of passage of the drugs in said compositions across body membranes.

In the cyclic ketone m + n is preferably from 11 to 15 and p is preferably 0. When R is alkyl it may be methyl, ethyl, propyl, isopropyl, butyl, sec-butyl, amyl, hexyl and the like. If the cyclic anhydrides (Formula II) m+n is preferably from 11 to 15, X is preferably 0, y is preferably 0 or 1 and z is preferably 0 or 1. In the cyclic esters, m+n is preferably from 11 to 15, x is preferably from 1 to 20, y is preferably from 1, and z is preferably 1. The drug composition which contains an effective amount of the desired active agent contains from about 0.1% to about 30% by weight of the selected lactone, cyclic ketone, or esters.

The drug composition, which may be administered topically, nasally, bucally, aurally, rectally, ocularly, orally, vaginally, or through the navel, may be in the form of solutions, creams, lotions, aerosols, suppositories or jellies; or incorporated in patches, films, or bandages.

The invention will become clearer from the examples which follow taken in conjunction with the drawings. These examples and drawings illustrate preferred embodiments of the invention and are not to be regarded as limiting.

The evaluation of the compositions of this invention in enhancing the rate of penetration of the drug through a body membrane was carried out in vitro using skin preparations obtained from homozygous Hr/Hr hairless mice (HRS/J) strain following the procedures described by Chow, Kaka and Wang in the J. Pharmaceut. Sci. 73 (12) 1794-1799 (1984) for the preparation, penetration study and data analysis.

Animals between 2 to 4 months of age were selected. In all selected animals the skins were grossly normal and free of bites, scratches or bruises. The mice were killed by $CO_2$ inhalation, and the skin was removed. The full-thickness skin was used in the penetration studies.

The skin preparation was mounted between the donor and receptor chambers of a Franz diffusion cell. The stratum corneum (SC) was exposed to the ambient condition and the dermal side was oriented toward a pH 7.4 saline-phosphate buffer, simulating the physiological pH of 7.3 - 7.4 of the dermal side, in the receptor chamber.

The solution of the receptor chamber was equilibrated by circulating water at 32°C through a jacket surrounding the chamber, which temperature was chosen to reflect the temperature of the SC, prior to the applications of the test sample. Mixing of the solution in the receptor chamber was accomplished by magnetic stirring.

A known amount of a radioisotope labeled drug, diluted with non-radioactive (cold) drug, with or without the adjuvant, was applied so as to spread across the SC surface of the mounted skin. Aliquots of the saline-phosphate buffer containing any radioisotope labeled drug which had penetrated through the skin into the receptor chamber were withdrawn from the side arm of the receptor chamber, and a volume of fresh saline-phosphate buffer equal to the volume of the withdrawn aliquot was added to the receptor chamber. Aliquots were withdrawn every 30 minutes during the first 2 hours and every hour during the next 10 hours, the total time of the study thus lasting up to 12 hours. The amount of the drug which had passed through the skin was measured by liquid scintillation counting of the withdrawn aliquot in Aquasol-2.

The drawings illustrate the penetration profile of the drugs. These profiles were constructed by plotting the amount of the drug which had penetrated the skin versus time. Profiles for control samples (no adjuvant added) and for tested samples (containing an adjuvant) were plotted in the same figure for purposes of comparison. The numbers of the figures correspond respectively to the numbers of the examples whose results they illustrate.

The permeability parameters which are shown in the tables were calculated in accordance with the method of Chow, Kaka and Wang as described on page 1795 of their paper.

Example 1

To a propylene glycol solution containing 4.74 x $10^{-2}$ mg/ml of tritiated triaminolone acetonide 2% w/v of the adjuvant was added. The adjuvants tested were 3-methylyclopentadecanone (I), cyclopentadecanone (II), cycloundecanone (III), and cyclododecanone (IV). Each of these cyclic ketones is commercially available. The preparations were tested according to the method described above, and the penetration profile of $H^3$ - triamcinolone acetonide as enhanced by each of these adjuvants is shown in figure 1, where each curve represents an average of the number of tests, N, carried with each adjuvant.

Based upon the data presented in figure 1, the total amount of tritiated triamcinolone acetonide and the rates of penetration (flux) calculated from the linear portion of the curve are shown in Table 1.

Table 1

| Adjuvant | Flux | | Total Amount* | |
|---|---|---|---|---|
| | X10$^3$dpm/cm$^2$/hr | Ratio % | dpm(X10$^3$) | Ratio % |
| Control | 0.16 | 100 | 1 | 100 |
| I | 0.70 | 437 | 3.5 | 350 |
| II | 1.07 | 669 | 4.8 | 480 |
| III | 0.25 | 156 | 1.5 | 150 |
| IV | 0.25 | 156 | 1.7 | 170 |

*Total amount of triamincinolone acetonide which penetrated at the end of 10 hours.

Example 2

The procedure of example 1 was repeated except that the only adjuvant tested was cyclopentadecanone at concentrations of 0.5, 1, 2, 3, 5 and 10% w/v. From 0.2 to 0.9 ml of methanol was added to 2.7 ml of the solution to help dissolve the ketone in the propylene glycol at higher concentrations. The presence of methanol did not appreciably change the permeability of the skin as demonstrated by the profile obtained with the control sample containing methanol. The penetration profiles are shown in figure 2, and it can be readily seen that the minimal effective concentration of the adjuvant was 2%.

Based upon the data presented in figure 2, the rates of flux calculated from the linear portion of the curve are given in Table 2.

Table 2

| Concentration of Adjuvant | Flux (dpm/cm$^2$/hr) | Ratio (%) |
|---|---|---|
| 10 | 7.4 x $10^3$ | 4625 |
| 5 | 4.1 x $10^3$ | 2563 |
| 3 | 3.7 x $10^3$ | 2310 |
| 2 | 3.7 x $10^3$ | 2310 |
| 1 | 0.31 x $10^3$ | 200 |
| 0.5 | 0.31 x $10^3$ | 200 |
| 0 (Control) | 0.16 x $10^3$ | 100 |

Example 3

The procedure of example 2 was repeated except that 3-methyl-cyclopentadecanone was used as the adjuvant and 0.1 to 0.3 ml ethanol was added to the solution to completely dissolve the adjuvant. This amount of ethanol did not appreciably change the permeability of the skin as demonstrated by the profiles of the controls with and without ethanol. The penetration profiles are shown in figure 3, and it can be readily seen that the minimal effective concentration of the adjuvant is 2%.

Based upon the data presented in figure 3, the rates of flux calculated from the linear portions of the curves are given in Table 3.

Table 3

| Concentration (%) | Flux (dpm/cm$^2$/hr) | Ratio (%) |
|---|---|---|
| 10 | $0.3 \times 10^3$ | 3000 |
| 5 | $0.3 \times 10^3$ | 3000 |
| 3 | $0.22 \times 10^3$ | 2200 |
| 2 | $0.15 \times 10^3$ | 1500 |
| 1 | $0.10 \times 10^3$ | 1000 |
| 0.5% | $0.013 \times 10^3$ | 130 |
| 0% (with ethanol) | $0.025 \times 10^3$ | 250 |
| 0% (no ethanol) | $0.010 \times 10^3$ | 100 |

Example 4

The procedure of example 1 was repeated except that the drug was 8-methoxy-psoralen (MOP) with a concentration of 46 mg/ml used as $H^3$-MOP dissolved in propylene glycol, and the adjuvants tested were 3-methylcyclopentadecanone (I) (0.4% w/v) and cycloundecanone (III) (2% w/v). The penetration profiles are shown in figure 4.

Based upon the data presented in figure 4, the rates of flux calculated from the lines portion of the curves are shown in Table 4.

Table 4

| Adjuvant | Flux (dpm/cm$^2$/hr) | Ratio (%) |
|---|---|---|
| Control | $1.88 \times 10^3$ | 100 |
| 0.4% I | $8.13 \times 10^3$ | 432 |
| 2% III | $3.63 \times 10^3$ | 193 |

Example 5

The process of example 1 was repeated except that tritiated clonidine, diluted 1000 fold with cold clonidine was used. The tests were run with a propylene glycol containing 37.4 mg/ml clonidine and 2% (w/v) cyclopentadecanone. The penetration profiles are shown in figure 5. Based on the profile the flux of the preparation containing the adjuvant was 10.1 mg/cm$^2$/hr or equivalent to $1.83 \times 10^6$ dpm/cm$^2$/hr of the respective radioisotopically labeled drug.

Example 6

The procedure of example 5 was repeated except that 14 C diazepam, diluted 100 fold with cold diazepam, was used. The tests were run with a propylene glycol solution containing 1.91 mg/ml of diazepam and 2% (w/v) cyclopentadecanone. The penetration profiles are shown in figure 6.

Example 7

The procedure of example 6 was repeated except that $^{14}$ C-diazepam diluted 1,000 fold with cold diazepam, was used. The propylene glycol solution contained 18.9 mg/ml of diazepam and 2% (w/v) of cyclopentadecanone. The penetration profiles are shown in figure 7.

Example 8

The procedure of example 6 was repeated except that $^{14}$ C estradiol, diluted 100 fold with cold estradiol, was used. The tests were run with a propylene glycol solution containing 1.06 mg/ml estradiol and 2% (w/v) cyclopentadecanone. The penetration profiles are shown in figure 8.

Example 9

The procedure of example 6 was repeated except that tritiated propranolol diluted 100 fold with cold propranolol, was used. The tests were run with a propylene glycol solution containing 9.7 x $10^{-3}$ mg/ml propranolol and 2% (w/v) cyclopentadecanone. The penetration profiles are showin in figure 9.

Example 10

The procedure of example 6 was repeated except that tritiated verapramil, diluted 1000 fold with cold verapramil, was used. The tests were run with a propylene glycol solution containing 1.54 x $10^{-2}$ mg/ml verapramil and 2% (w/v) cyclopentadecanone. The penetration profiles are shown in figure 10.

The results of the experiments described in examples 1 to 10 clearly show that the cyclic ketones of the formula described above enhance the rate of transdermal passage of large variety of drugs. These drugs include steroids (estradiol and triamcinolone acetate), antihypertensives (clonidine and verapramil), sedatives (diazepam), and antiarrhythmics (propranolol). Other types of drugs whose rate of transdermal passage would be increased include, but are not limited to, antibiotics, antifungal agents, CNS depressants, and sunscreens.

Examples 1 to 13 have shown solutions containing compositions which are suitable in the practice of this invention. Examples 14 to 18 illustrate other types of compositions which are also suitable. In these examples the amounts are given in percent by weight.

Studies were carried out to demonstrate that: (1) the cyclic ketones containing more than 10 carbon atoms possess unexpected, desirable properties not possessed by those ketones having a lower carbon content; (2) other macrocyclic compounds such as cyclopentadecanolide (having an oxygen in the macrocyclic ring) and civetone (having a double bond in the macrocyclic ring) possess properties which enhance the skin absorption of drugs through skin; and (3) nasal absorption of drugs, in particular therapeutic proteins and peptides, can be enhanced by the addition of such macrocyclic compounds. These studies are described in Examples 11 to 13.

Example 11

Comparison of different cyclic ketones for the enhancement of percutaneous absorption of drugs through hairless mouse skin

In this study, six different cyclic ketones were used for comparative studies on the percutaneous absorption of tritiated hydrocortisones through hairless mouse skin. These included cyclononanone (C9), cyclodecanone (C10), cycloundecanone (C11), cyclododecanone (C12), cyclotridecanone (C13), and cyclopentadecanone (C15). The preparation, penetration study, and data analysis of the experiment followed the procedure referred to in Example 1. For each compound, five skin samples were used for percutaneous absorption study. The concentration of enhancers used in the donor compartment was 2%. The duration of the experiment was performed for 10 hours when the steady-rate of penetration of drugs has been reached for at least several hours. Figure 11 shows penetration profiles of hydrocortisone from percutaneous absorption enhanced by the different cyclic ketones through hairless mouse skin. The ranking of the potency of the enhanced absorption property of different cyclic ketones are in the following order: cyclopentadecanone > cyclotridecanone > cyclododecanone > cyclononanone > cycloundecanone > cyclodecanone (a decreasing order). The slope of the penetration profiles, which represent the steady state permeation rate of drugs, were calculated and shown in Table 5. The enhancement factor of different cyclic ketones was calculated based upon the control group as 100. There was a slight decrease in the permeation rate of hydrocortisone through hairless mouse skin when cyclodecanone and cycloundecanone

were used as skin enhancers respectively. In other words, both cyclodecanone and cycloundecanone slightly inhibit the percutaneous absorption of hydrocortisone through hairless mouse skin. There was a little effect in the percutaneous absorption of hydrocortisone through hairless mouse skin when cyclononanone was used. There was a 230% increase in the permeation rate of hydrocortisone through hairless mouse skin when cyclododecanone was used in the study. However, there was a 524% increase and a 590% increase in percutaneous absorption of hydrocortisone through hairless mouse skin when cyclotridecanone and cyclopentadecanone were used as skin enhancers respectively. Additionally, cyclopentadecanolide, a macrocyclic compound having an oxygen atom in the macrocyclic ring, was used in the same study for comparison. There was a 17-fold increase in percutaneous permeation rate of hydrocortisone through hairless mouse skin.

From this study, it was clearly demonstrated that (1) the cyclic ketones containing more than 11 carbon atoms possess unexpected, desirable properties which are not possessed by those ketones having a lower carbon content, (2) the higher the carbon number in the macrocyclic ring, the higher the enhanced permeation rate of hydrocortisone through hairless mouse skin, and (3) the cyclopentadecanolide is superior to cyclic ketones being tested in this study.

Table 5

| Comparison of permeation rate of hydrocortisone through hairless mouse skin by different cyclic ketones | | |
|---|---|---|
| Chemical(s) | Permeation Rate (ug/cm*cm/hr) | Enhancement factor (%) |
| None or control | $5.25 \times 10^{-5}$ | 100 |
| cyclononanone | $5.96 \times 10^{-5}$ | 113 |
| cyclodecanone | $3.79 \times 10^{-5}$ | 72 |
| cycloundecanone | $3.91 \times 10^{-5}$ | 74 |
| cyclododecanone | $1.21 \times 10^{-4}$ | 230 |
| cyclotridecanone | $2.75 \times 10^{-4}$ | 524 |
| cyclopentadecanone | $3.10 \times 10^{-4}$ | 590 |
| cyclopentadecanolide | $8.94 \times 10^{-4}$ | 1703 |
| 1. The concentration of chemical used in the donor compartment was 2%.<br>2. Permeation rates were calculated from the slope of permeation profile.<br>3. The enhancement factor was calculated based upon the control group (without chemical) as 100. | | |

<u>Example 12</u>

Macrocyclic compounds other than cyclic ketones

A. Civetone, 9-cycloheptadecen-1-one.

Sample preparation, permeation study and data analysis were carried out following the procedure referred to in Example 1. The enhancer used in this study is civetone at the level of 2% in the solution of donor compartment of diffusion cell.

Figure 12 shows the permeation profile of tritiated triamcinolone acetonide through hairless mouse skin with and without civetone. the steady-rate permeation rate, calculated from the slope of permeation profile, was $8.36 \times 10^{-3}$ ug/cm*cm/hr with civetone; while it is only $1.10 \times 10^{-3}$ ug/cm *cm/hr without civetone. There was a 760% increase in the percutaneous permeation rate of triamcinolone acetonide when civetone was used as skin enhancer at the level of 2%.

B. Cyclopentadecanolide

Sample preparation, permeation study and data analysis were carried out using the same procedures as in Part A, above, except cyclopentadecanolide instead of civetone was used.

Figure 13 shows the permeation profiles of tritiated triamcinolone acetonide with cyclopentadecanolide. Without the addition of cyclopentadecanolide, no penetrated drug was detected in the receptor compartment. However, when cyclopentadecanolide was used at the level of 2%, the drug, triamcinolone acetonide

penetrated through hairless mouse skin. From the permeation profile, four permeation parameters, i.e., lag time, permeability coefficient of membrane (Kp), diffusion constant within membrane (D), and partition coefficient between membrane and vehicle (Km) were analyzed and listed in Table 6.

Table 6

| Triamcinolone acetonide penetration parameters with and without cyclopentadecanolide | | | | |
|---|---|---|---|---|
| Enhancer | Lag time$_{(hr)}$ | Kp (cm/hr) | D ($cm^2$/hr) | Km |
| None | -- | -- | -- | -- |
| cyclopentadecanolide (2%) | 6.03 | 3.88 | 4.42 x $10^{-7}$ | 3.51 x $10^4$ |

Example 13

Nasal absorption of insulin in dogs

A. Cyclopentadecanolide (or oxacyclohexadecan-2-one)

The object of this study was to demonstrate the nasal absorption of therapeutic proteins and peptides, carbohydrates, nucleic acids, lipoproteins, mucoproteins, lipoproteins, and other macromolecules in living animals and humans can be achieved with the addition of skin enhancers such as cyclopentadecanolide.

Beagle dogs weighing 10 to 12 kg were used in this study. The formulation of the nasal spray was composed of Freon, insulin, and cyclopentadecanolide packaged in a metered nasal spray device which is commercially available. Before applying nasal spray in dogs, dogs were anaesthesized using Nembutal (or pentabarbitol) at the dose of 40-50 mg/kg. Fifteen minutes before application, blood samples were obtained. Then, nasal spray of insulin was applied with the aid of applicator. Blood samples were again obtained at 0, 10, 20, 30, 45, 60, 90, 120, and 180 minutes. Both blood glucose determined by YSI glucose analyser and serum insulin levels determined by radioimmunoassay were tested. Both methods were commonly practiced in the laboratory.

Table 7 shows the blood glucose and serum insulin levels of dogs receiving insulin nasal spray containing cyclopentadecanolide. Obviously, when nasal spray of insulin with cyclopentadecanolide was applied (sprayed) in the nasal cavity of dogs, serum insulin levels abruptly increased to 71.2 uU/ml in 10 minutes and maintained the level for about 30 minutes, then gradually decreased and levelled off in 3 hours. On the other hand, blood glucose levels decreased from 83.6 mg/dl at 0 minute to 51.5 mg/di at 30 minutes as serum insulin levels increased from 2.7 uU/ml at 0 minute to 67.1 uU/ml at 30 minutes. Then, the blood glucose levels maintained almost constant for about 80 minutes. Finally, when serum insulin was depleting at 120 minutes to 7.9 uU/ml at 180 minutes, blood glucose levels rose from 45.8 mg/dl to 72.7 mg/dl within the same time span.

Figure 14 shows the time course of both blood glucose and serum insulin levels in dogs before and after receiving nasal spray of insulin containing cyclopentadecanolide. These patterns were similar to those receiving insulin subcutaneously.

Table 7

| Nasal Absorption of Insulin in Dogs with Cyclopentadecanolide | | |
|---|---|---|
| Time (minutes) | Blood Glucose (mg/dl) | Serum Insulin (uU/ml) |
| - 15 | 81.0 ± 3.2 | 1.7 ± 0.6 |
| 0 | 83.6 ± 1.6 | 2.7 ± 1.3 |
| 10 | 80.7 ± 2.7 | 71.2 ± 28.3 |
| 20 | 68.4 ± 9.1 | 78.6 ± 26.6 |
| 30 | 51.5 ± 9.5 | 67.1 ± 23.9 |
| 45 | 35.2 ± 6.6 | 53.3 ± 13.6 |
| 60 | 40.1 ± 5.3 | 40.7 ± 10.9 |
| 90 | 38.7 ± 0.4 | 14.2 ± 3.9 |
| 120 | 45.8 ± 3.0 | 10.8 ± 2.7 |
| 180 | 72.7 ± 8.3 | 7.9 ± 2.8 |
| 1. Three dogs were used in the study<br>2. Data were expressed as mean ± S.E.M.<br>3. The dose of insulin used in each dog was 1 U/kg body weight<br>4. The concentration of cyclopentadecanolide in Freon solution was 1%. | | |

Control experiments included the following: (1) Placebo without insulin but containing skin enhancer, (2) Phosphate buffer solution, and (3) Insulin itself. When these control formulations were sprayed in the nasal cavity in dogs, no changes in both blood glucose level and serum insulin were found.

B. 3-methyl cyclopentadecanone

In a separate study, 3-methyl cyclopentadecanone (musone) instead of cyclopentadecanolide, was used as enhancer for nasal absorption of insulin. The formulation of nasal spray was the same as the previous example except the enhancer used in the formulation. The procedures and the methods for performing the experiment were the same as previous example. Blood samples were asssayed for blood glucose and serum insulin levels at given time intervals. Two dogs were used in this study. The average values of blood glucose and serum insulin were shown in Table 8. And the time course of the changes of blood glucose and serum insulin levels were shown in Figure 15. From this study, it can be concluded that the effect of 3-methyl cyclopentadecanone on the nasal absorption of insulin in dogs was similar to that of cyclopentadecanolide used in the nasal spray formulation.

Table 8

| Nasal Absorption of Insulin in Dogs with 3-methyl cyclopentadecanone | | |
|---|---|---|
| Time (minute) | Glucose level (mg/dl) | Serum insulin (uU/ml) |
| - 20 | 92.1 ± 0.6 | 7.9 ± 0.9 |
| 0 | 96.4 ± 5.0 | 15.8 ± 6.7 |
| 10 | 92.1 ± 3.2 | 40.5 ± 8.9 |
| 20 | 84.7 ± 0.9 | 40.3 ± 4.1 |
| 30 | 71.3 ± 2.6 | 36.5 ± 10.0 |
| 60 | 50.4 ± 10.1 | 45.9 ± 13.7 |
| 90 | 41.0 - 9.7 | 24.1 ± 1.6 |
| 120 | 39.3 ± 5.6 | 23.5 ± 3.0 |
| 180 | 64.5 ± 24.4 | 22.5 ± 3.7 |
| 1. Two dogs were used in the study<br>2. Data were expressed as mean ± S.E.M.<br>3. The dose of insulin used in each dog was 1 U/kg body weight<br>4. The concentration of 3-methyl cyclopentadecanone in Freon solution was 1%. | | |

Examples 1 to 13 have shown solutions containing compositions which are suitable in the practice of this invention. In particular, example 13 illustrates the use of macrocyclic compounds in the nasal spray of insulin formulations for diabetes treatment. The practice of this invention is not limited to insulin alone, but suitable for many therapeutic proteins and peptides. To name a few, interferon for common colds, cancer, and viral infection, lymphokines for cancer and immunity disease, growth hormones for drawfism, lutenizing hormone releasing hormones (LHRH) analogs for birth control, enkaphalin for pain relief, and so on. Examples 14 to 18 illustrate other types of compositions which are also suitable. In these examples the amounts are given in percent by weight.

Example 14

The following lotion formulation containing from about 0.001 to 1% by weight of estradiol may be prepared:

| | |
|---|---|
| Estradiol | 0.001-1 |
| Cetylalcohol | 15 |
| Propyleneglycol | 10 |
| Sodium lauryl sulfate | 15 |
| Cyclopentadecanone | 2 |
| Water | q.s. 100 |

Example 15

The following cream formulation containing clotrimazole, an antifungal agent, may be prepared:

| | |
|---|---|
| Mineral oil | 31 |
| Cyclopentadecanone | 2 |
| Clotrimazole | 1 |
| Spermaceti | 10 |
| Glycerol monostearate | 10 |
| Paraffin | 8 |
| Water | 38 |

Example 16

The following suppository containing an antiseptic, benzethonium chloride, may be prepared:

| | |
|---|---|
| Benzethonium chloride | 2 |
| Cyclopentadecanone | 2 |
| Cocoa butter | 80 |
| Tween 61* | 16 |

*Polyethylene - 4 - sorbitan monostearate

Example 17

The following film containing procaine hydrochloride may be prepared:

| | |
|---|---|
| Procaine hydrochloride | 0.562 |
| Cyclopentadecanone | 2 |
| Polyvinyl alcohol | 30 |
| Polyvinylpyrrolidone | 30 |
| Polyethylene glycol | q.s. 100 |

Example 18

Vaginal Absorption of Fluorogestone Acetate for Estrus Synchronization in Sheep .

The objective of this study was to demonstrate the vaginal absorption of therapeutic agents can be achieved to desirable therapeutic levels by the addition of permeation enhancers such as cyclopentadecanolide. Polymer sponges made of polyurethane or alike are impregnated with 80% fluorogestone acetate and 20% cyclopentadecanolide. The sponge was inserted into the vagina of ewes for up to 12 days. Blood samples were drawn and the levels of fluorogestone acetate were determined by radioimmunoassay. Table 9 shows the blood levels of fluorogestone acetate in ewes during the time course of treatment. The later phase of treatment is the decisive indicator for estrus synchronization in ewes. The results clearly indicated that at the later phase of treatment (i.e. days 6, 9, and 12), the blood levels in those ewes receiving sponges containing permeation enhancers such as cyclopentadecanolide are higher than those without permeation enhancers.

| Treatment and Animal No. | | Day of Treatment | | | | | |
|---|---|---|---|---|---|---|---|
| | | 0 | 3 | 6 | 9 | 12 | 13 |
| | | (Nanogram/ml) | | | | | |
| -ve | 1 | 3.61 | 0.19 | 0.47 | 0.22 | 0.20 | 0.34 |
| Control | 2 | 6.82 | 0.48 | 0.39 | 0.25 | 0.19 | 0.23 |
| | 3 | 0.69 | 0.36 | 0.36 | 0.07 | 0.09 | 0.32 |
| | $\overline{X}$ | 3.70 | 0.34 | 0.41 | 0.18 | 0.16 | 0.30 |
| | SED | 1.77 | 0.08 | 0.03 | 0.05 | 0.03 | 0.03 |
| Sponge I | 4 | 0.56 | 2.61 | 1.42 | 2.05 | 1.11 | 0.39 |
| (No | 5 | 3.15 | 3.23 | 2.26 | 1.49 | 1.56 | 0.19 |
| Enhancer) | 6 | 5.51 | 3.26 | 3.61 | 2.53 | 2.41 | 0.43 |
| | 7 | 0.80 | 2.06 | 1.39 | 2.05 | 1.47 | 0.22 |
| | $\overline{X}$ | 2.51 | 2.79 | 2.17 | 2.03 | 1.64 | 0.31 |
| | SED | 1.16 | 0.28 | 0.52 | 0.21 | 0.28 | 0.06 |
| Sponge II | 8 | 2.62 | 2.12 | 2.06 | 3.61 | 2.51 | 0.34 |
| (With | 9 | 0.87 | 4.27 | 2.53 | 2.31 | 2.13 | 0.41 |
| Enhancer) | 10 | 0.82 | 3.33 | 2.18 | 2.39 | 2.04 | 0.59 |
| | 11 | 1.06 | 2.02 | 2.22 | 2.81 | 2.24 | 0.63 |
| | $\overline{X}$ | 1.34 | 2.94 | 2.56 | 2.78 | 2.23 | 0.49 |
| | SED | 0.43 | 0.54 | 0.10 | 0.30 | 0.10 | 0.07 |

## Claims

**1.** A composition for administering a physiologically active agent across skin or a body membrane of an animal or human which contains an effective amount of the active agent and from about 0.1% to about 30% by weight of a lactone or a cyclic ketone of the formula (1) or a cyclic monoester or diester, or a cyclic ether-ester or a cyclic keto-ester of the formula (II)

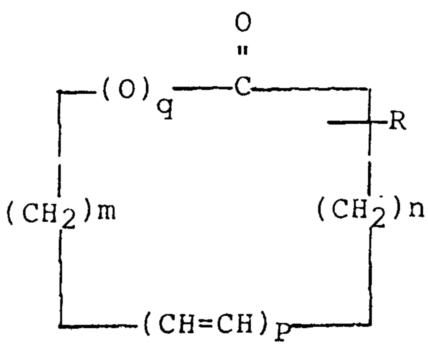

Formula I

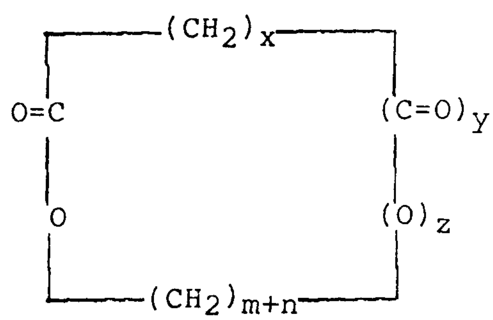

Formula II

wherein m and n are integers having a value from 1 to 20 with the proviso that m + n is at least 11 and not greater than 25, p is an integer having a value of 0 or 1, q is an integer having a value of 0 or 1, and R is hydrogen or an alkyl group having from 1 to 6 carbon atoms, x is an integer having a value of 0 or 1, y is an integer having a value of 0 or 1, and z is an integer having a value of 0 or 1.

**2.** A composition according to claim 1 wherein q is 0.

**3.** A composition according to claim 1 or 2 wherein p is 0.

**4.** A composition according to any one of claims 1 to 3 wherein m + n is an iteger having a value from 11 to 15.

**5.** A composition according to claim 4 wherein m + n is 11 or 14.

**6.** A composition according to claim 5 wherein m + n is 14 and R is methyl.

**7.** A composition according to any one of claims 1 to 5 wherein R is hydrogen.

**8.** A composition according to claim 1 or 2 wherein p is 1, m is 7 and n is 7.

**9.** A composition according to claim 1 wherein p is 0, q is 1, and m + n is 15.

**10.** A composition according to any one of claims 1 to 9 which is in the form of a solution.

**11.** A composition according to any one of claims 1 to 10 wherein the concentration of the cyclic ketone is at least about 2%.

**12.** A composition according to claim 11 wherein the concentration of macrocyclic lactone is at least about 0.5%.

**13.** A composition according to claim 12 wherein the concentration of macrocyclic lactone is at least about 0.1%.

**14.** A composition according to any one of claims 1 to 13 which is impregnated in the form of a sponge or is in the form of an aerosol spray.

**Revendications**

**1.** Une composition pour administrer un agent physiologiquement actif à travers la peau ou une membrane corporelle d'un animal ou d'un être humain, qui contient une quantité efficace de l'agent actif et d'environ O,1 % à environ 30 % en poids d'un lactone ou d'un cétone cyclique de formule (I) ou d'un mono- ou diester cyclique ou d'un éther-ester cyclique ou d'un cétone-ester cyclique de formule (II)

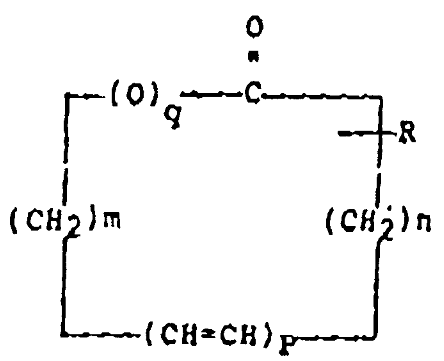

formule I

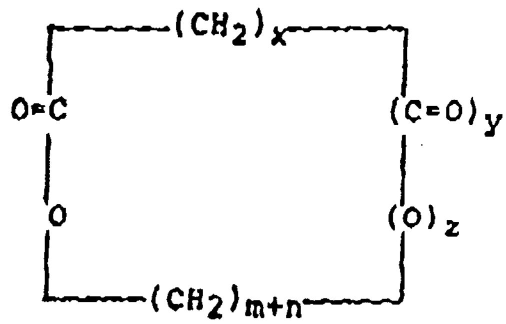

formule II

dans lesquelles m et n sont des entiers ayant une valeur comprise entre 1 et 20, sous réserve que m + n est au moins égal à 11 et n'est pas supérieur à 25, p est un entier ayant comme valeur O ou 1, q est un entier ayant comme valeur O ou 1, et R est l'hydrogène ou un groupe alkyle ayant de 1 à 6 atomes de c4arbone, x est un entier ayant comme valeur O ou 1, y est un entier ayant comme valeur O ou 1, et z est un entier ayant comme valeur O ou 1.

**2.** Une composition selon la revendication 1, dans laquelle q est égal à O.

**3.** Une composition selon l'une des revendications 1 ou 2, dans laquelle p est égal à O.

**4.** Une composition selon l'une des revendications 1 à 3, dans laquelle m + n est un entier ayant une

valeur comprise entre 11 et 15.

**5.** Une composition selon la revendication 4, dans laquelle m + n est égal à 11 ou 14.

**6.** Une composition selon la revendication 5, dans laquelle m + n est égal à 14 et R est méthyle.

**7.** Une composition selon l'une des revendications 1 à 5, dans laquelle R est l'hydrogène.

**8.** Une composition selon l'une des revendications 1 ou 2, dans laquelle p est égal à 1, m est égal à 7 et n est égal à 7.

**9.** Une composition selon la revendication 1, dans laquelle p est égal à O, q est égal à 1 et m + n est égal à 15.

**10.** Une composition selon l'une des revendications 1 à 9, qui est sous la forme d'une solution.

**11.** Une composition selon l'une des revendications 1 à 10, dans laquelle la concentration de cétone cyclique est au moins d'environ 2 %.

**12.** Une composition selon la revendication 11, dans laquelle la concentration de lactone macrocyclique est au moins d'environ 0,5 %.

**13.** Une composition selon la revendication 12, dans laquelle la concentration de lactone macrocyclique est au moins d'environ O,1 %.

**14.** Une composition selon l'une des revendications 1 à 13, qui est imprégnée sous forme d'une éponge ou est sous forme d'une pulvérisation aérosol.

**Patentansprüche**

**1.** Präparat zur Verabreichung eines physiologisch aktiven Mittels durch die Haut oder eine Membran eines tierischen oder menschlichen Körpers, enthaltend eine wirksame Menge des aktiven Mittels und von etwa 0,1% bis etwa 30 Gew.-% eines Lactons oder eines cyclischen Ketons der Formel (I) oder eines cyclischen Monoesters oder Diesters oder eines cyclischen Ether-Esters oder eines cyclischen Ketoesters der Formel (II)

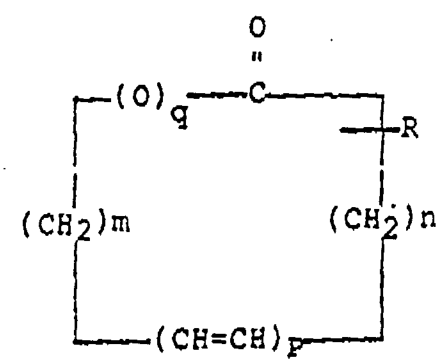

Formel I

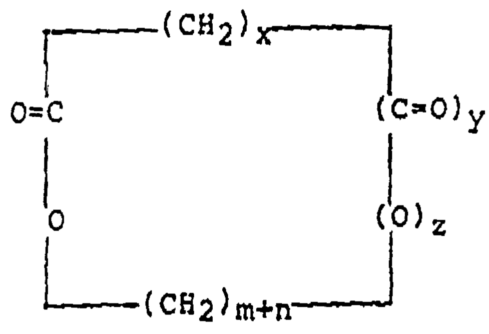

Formel II

wobei m und n ganze Zahlen mit einem Wert von 1 bis 20 sind, mit der Maßgabe, daß m+n mindestens 11 und nicht mehr als 25 beträgt, p eine ganze Zahl mit dem Wert 0 oder 1 ist, q eine ganze Zahl mit dem Wert 0 oder 1 ist, und R ein Wasserstoffatom oder einen Alkylrest mit 1 bis 6 Kohlenstoffatomen bedeutet, x eine ganze Zahl mit einem Wert von 0 oder 1 ist, y eine ganze Zahl mit einem Wert von 0 oder 1 ist und z eine ganze Zahl mit einem Wert von 0 oder 1 ist.

**2.** Präparat nach Anspruch 1, dadurch **gekennzeichnet,** daß q für 0 steht.

3. Präparat nach Anspruch 1 oder 2, dadurch **gekennzeichnet,** daß p für 0 steht.

4. Präparat nach einem der Ansprüche 1 bis 3, dadurch **gekennzeichnet,** daß m + n eine ganze Zahl mit einem Wert von 11 bis 15 ist.

5. Präparat nach Anspruch 4, dadurch **gekennzeichnet,** daß m + n den Wert 11 oder 14 hat.

6. Präparat nach Anspruch 5, dadurch **gekennzeichnet,** daß m + n den Wert 14 hat und R für Methyl steht.

7. Präparat nach einem der Ansprüche 1 bis 5, dadurch **gekennzeichnet,** daß R für ein Wasserstoffatom steht.

8. Präparat nach Anspruch 1 oder 2, dadurch **gekennzeichnet,** daß p für 1 steht, m für 7 steht und n für 7 steht.

9. Präparat nach Anspruch 1, dadurch **gekennzeichnet,** daß p für 0 steht, q für 1 steht und m + n den Wert 15 hat.

10. Präparat nach einem der Ansprüche 1 bis 9, dadurch **gekennzeichnet,** daß es in Form einer Lösung vorliegt.

11. Präparat nach einem der Ansprüche 1 bis 10, dadurch **gekennzeichnet,** daß die Konzentration des cyclischen Ketons mindestens etwa 2% beträgt.

12. Präparat nach Anspruch 11, dadurch **gekennzeichnet,** daß die Konzentration des makrocyclischen Lactons mindestens etwa 0,5% beträgt.

13. Präparat nach Anspruch 12, dadurch **gekennzeichnet,** daß die Konzentration des makrocyclischen Lactons mindestens etwa 0,1% beträgt.

14. Präparat nach einem der Ansprüche 1 bis 13, dadurch **gekennzeichnet,** daß es in impregnierter Form als Schwamm oder in der Form eines Aerosolsprays vorliegt.

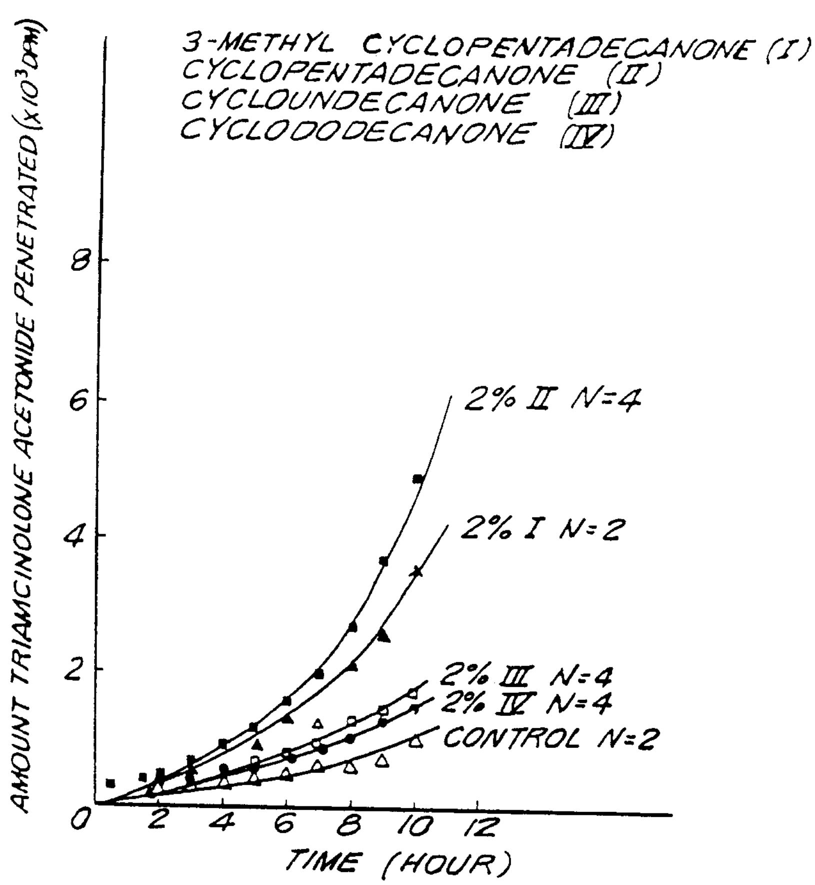
3-METHYL CYCLOPENTADECANONE (I)
CYCLOPENTADECANONE (II)
CYCLOUNDECANONE (III)
CYCLODODECANONE (IV)
AMOUNT TRIAMCINOLONE ACETONIDE PENETRATED (x10³ DPM)
2% II N=4
2% I N=2
2% III N=4
2% IV N=4
CONTROL N=2
0 2 4 6 8 10 12
TIME (HOUR)

FIG.1

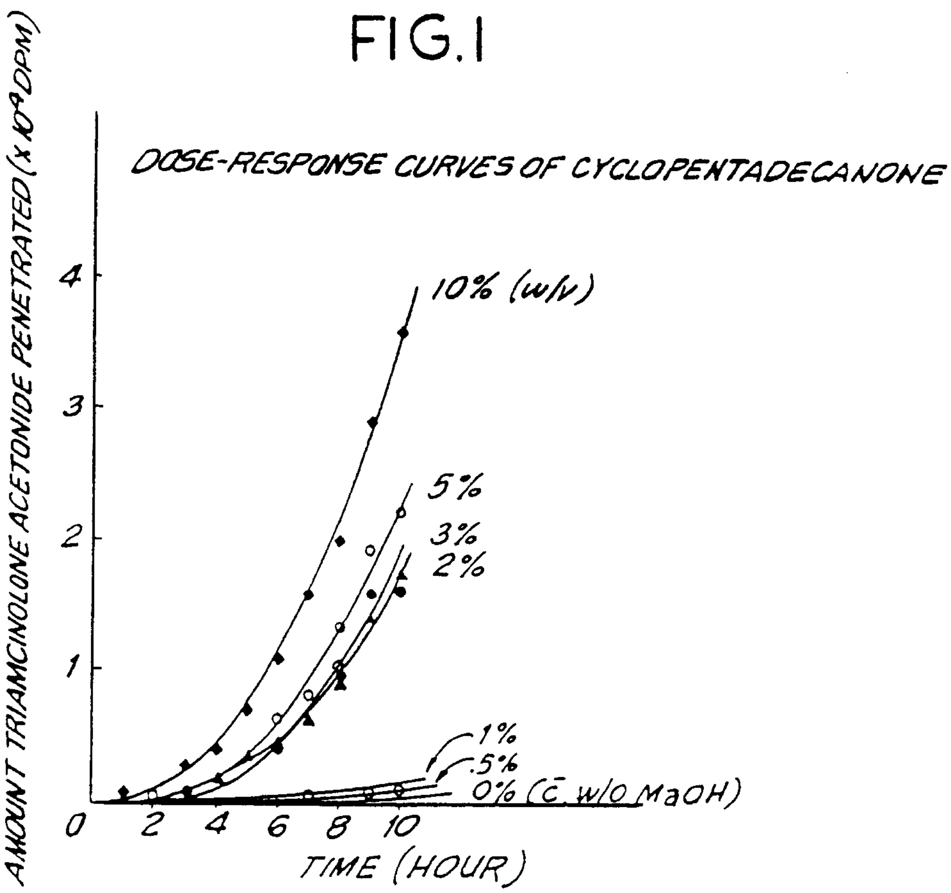
DOSE-RESPONSE CURVES OF CYCLOPENTADECANONE
AMOUNT TRIAMCINOLONE ACETONIDE PENETRATED (x 10⁴ DPM)
10% (w/v)
5%
3%
2%
1%
.5%
0% (C̄ w/o MaOH)
0 2 4 6 8 10
TIME (HOUR)

FIG.2

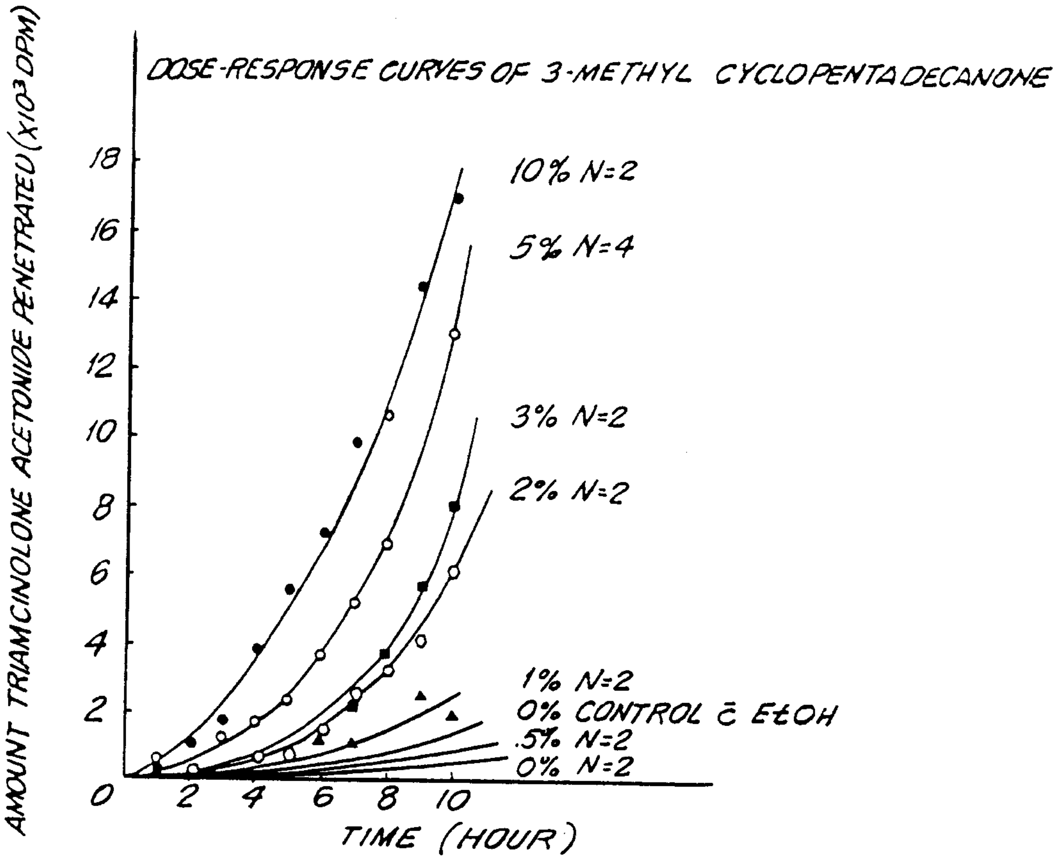
DOSE-RESPONSE CURVES OF 3-METHYL CYCLOPENTADECANONE
AMOUNT TRIAMCINOLONE ACETONIDE PENETRATED ($\times 10^3$ DPM)
18
16
14
12
10
8
6
4
2
0
2
4
6
8
10
TIME (HOUR)
10% N=2
5% N=4
3% N=2
2% N=2
1% N=2
0% CONTROL c̄ EtOH
.5% N=2
0% N=2

FIG.3

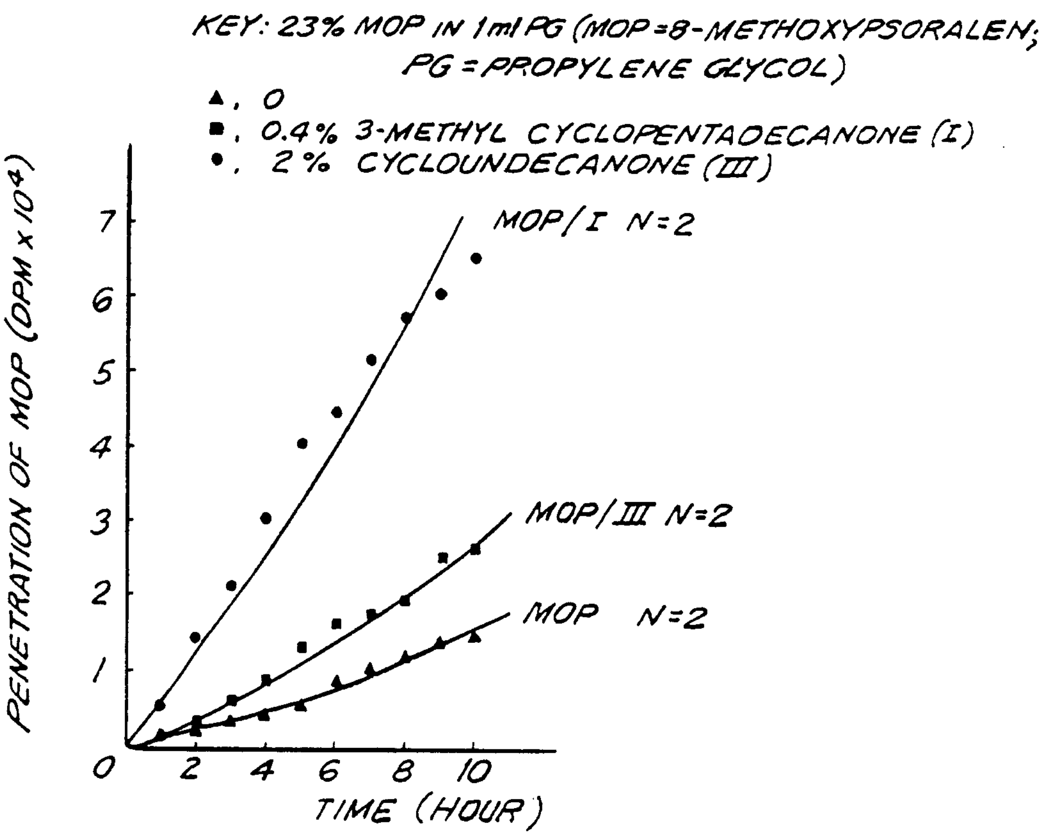
KEY: 23% MOP IN 1ml PG (MOP=8-METHOXYPSORALEN; PG=PROPYLENE GLYCOL)
▲, 0
■, 0.4% 3-METHYL CYCLOPENTADECANONE (I)
●, 2% CYCLOUNDECANONE (III)
PENETRATION OF MOP (DPM $\times 10^4$)
7
6
5
4
3
2
1
0
2
4
6
8
10
TIME (HOUR)
MOP/I N=2
MOP/III N=2
MOP N=2

FIG.4

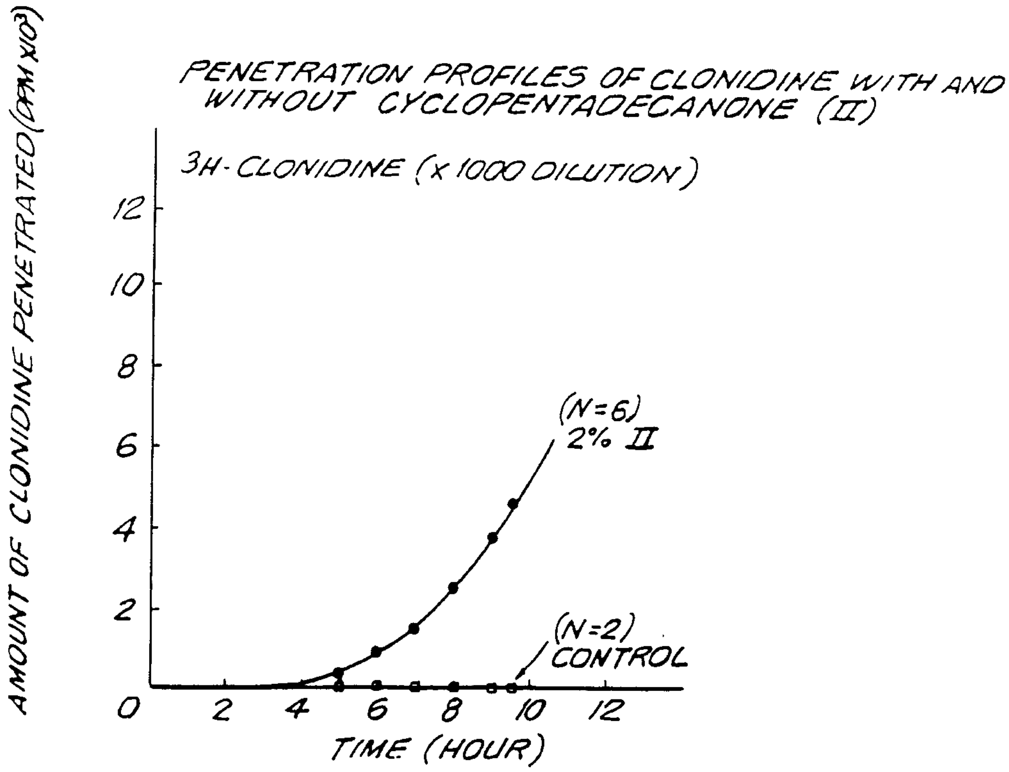
PENETRATION PROFILES OF CLONIDINE WITH AND WITHOUT CYCLOPENTADECANONE (II)
3H-CLONIDINE (x 1000 DILUTION)
AMOUNT OF CLONIDINE PENETRATED (DPM x10³)
(N=6)
2% II
(N=2)
CONTROL
TIME (HOUR)
0
2
4
6
8
10
12

FIG.5

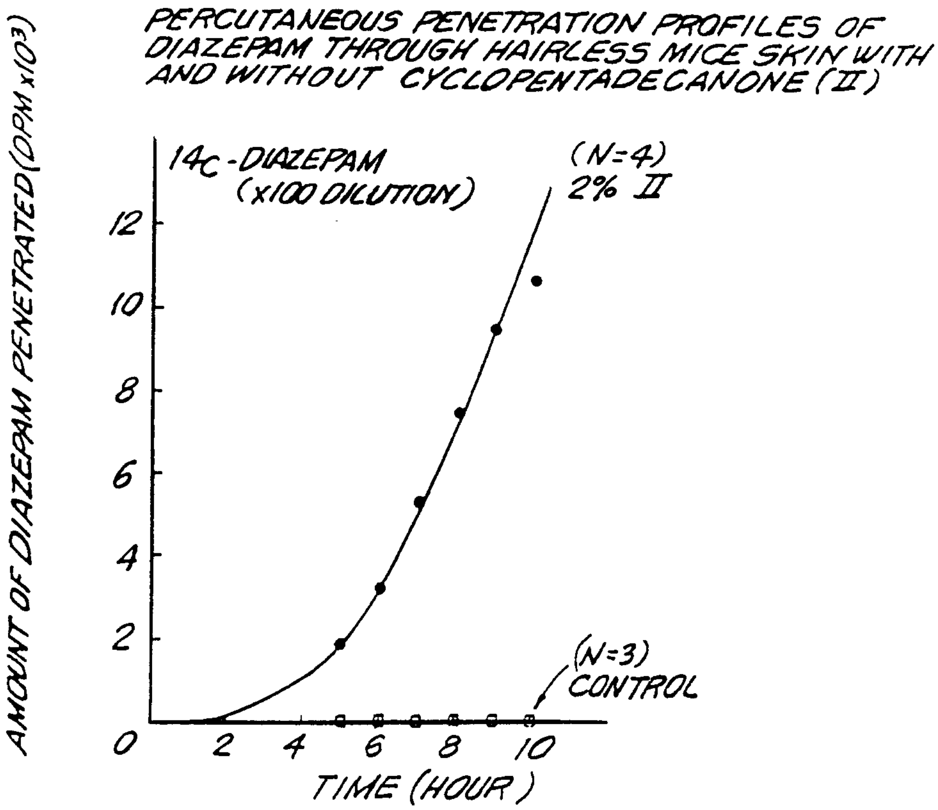
PERCUTANEOUS PENETRATION PROFILES OF DIAZEPAM THROUGH HAIRLESS MICE SKIN WITH AND WITHOUT CYCLOPENTADECANONE (II)
14C-DIAZEPAM
(x100 DILUTION)
AMOUNT OF DIAZEPAM PENETRATED (DPM x10³)
(N=4)
2% II
(N=3)
CONTROL
TIME (HOUR)
0
2
4
6
8
10
12

FIG.6

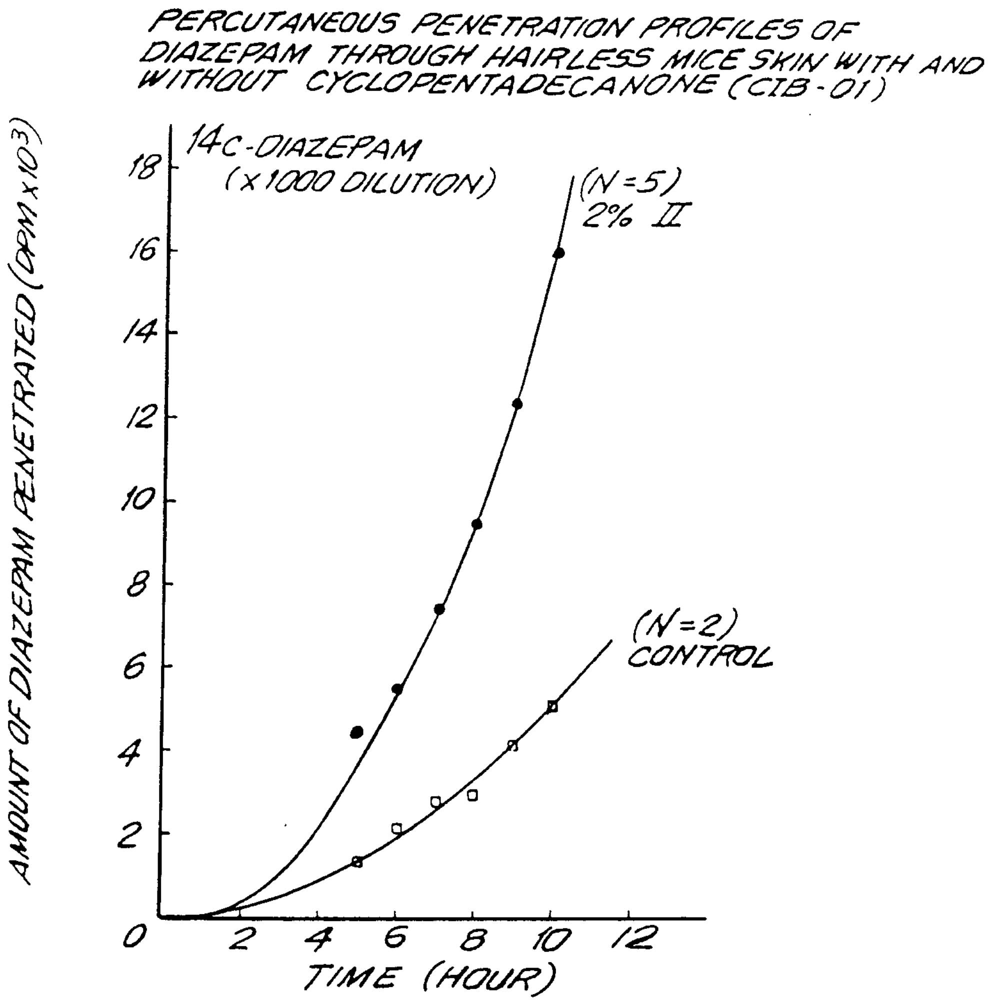
PERCUTANEOUS PENETRATION PROFILES OF DIAZEPAM THROUGH HAIRLESS MICE SKIN WITH AND WITHOUT CYCLOPENTADECANONE (CIB-01)
14C-DIAZEPAM (x1000 DILUTION)
(N=5) 2% II
(N=2) CONTROL
AMOUNT OF DIAZEPAM PENETRATED (DPM x 10³)
TIME (HOUR)

FIG.7

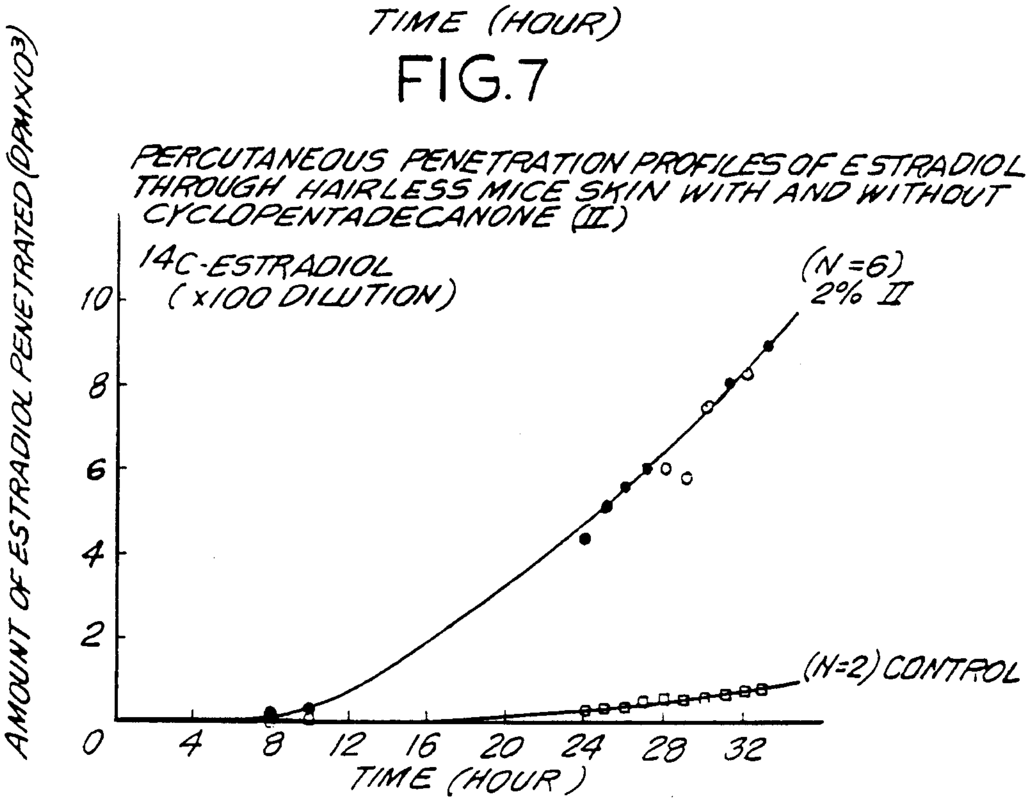
PERCUTANEOUS PENETRATION PROFILES OF ESTRADIOL THROUGH HAIRLESS MICE SKIN WITH AND WITHOUT CYCLOPENTADECANONE (II)
14C-ESTRADIOL (x100 DILUTION)
(N=6) 2% II
(N=2) CONTROL
AMOUNT OF ESTRADIOL PENETRATED (DPM x 10³)
TIME (HOUR)

FIG.8

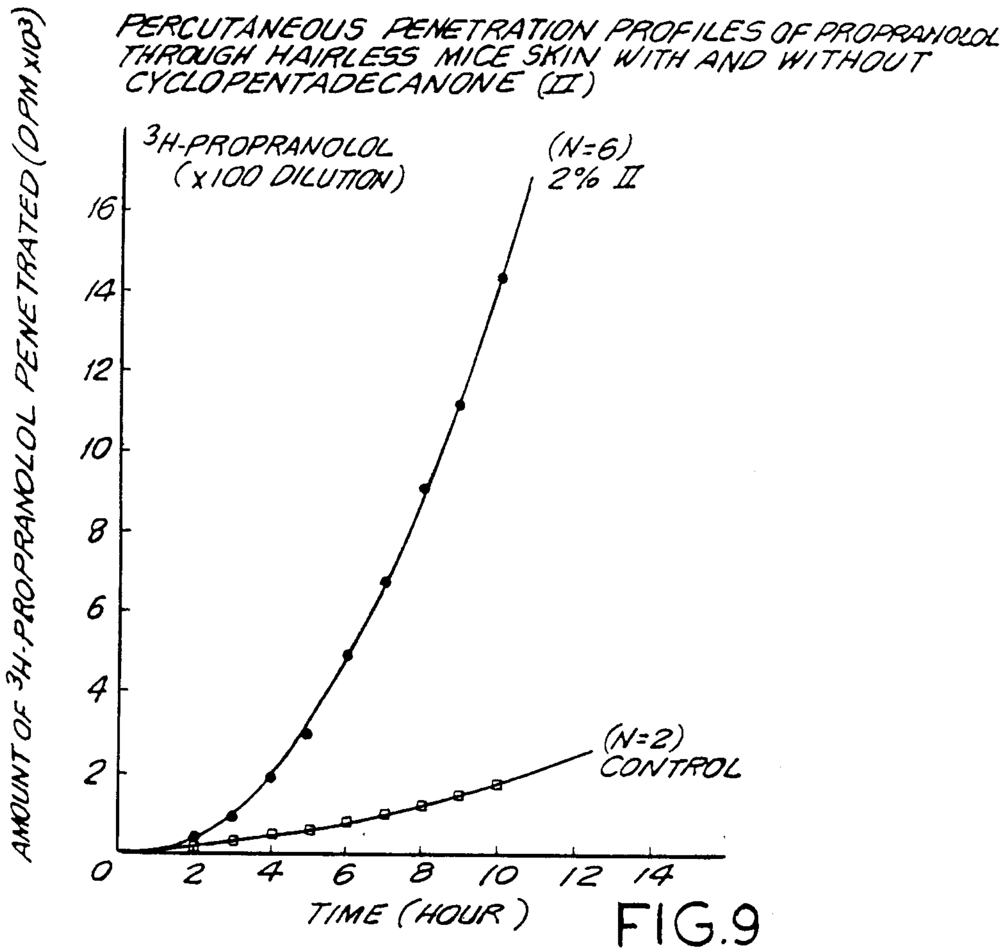
PERCUTANEOUS PENETRATION PROFILES OF PROPRANOLOL THROUGH HAIRLESS MICE SKIN WITH AND WITHOUT CYCLOPENTADECANONE (II)
3H-PROPRANOLOL
(x100 DILUTION)
(N=6)
2% II
(N=2)
CONTROL
AMOUNT OF 3H-PROPRANOLOL PENETRATED (DPM x10³)
TIME (HOUR)
0
2
4
6
8
10
12
14
16

FIG.9

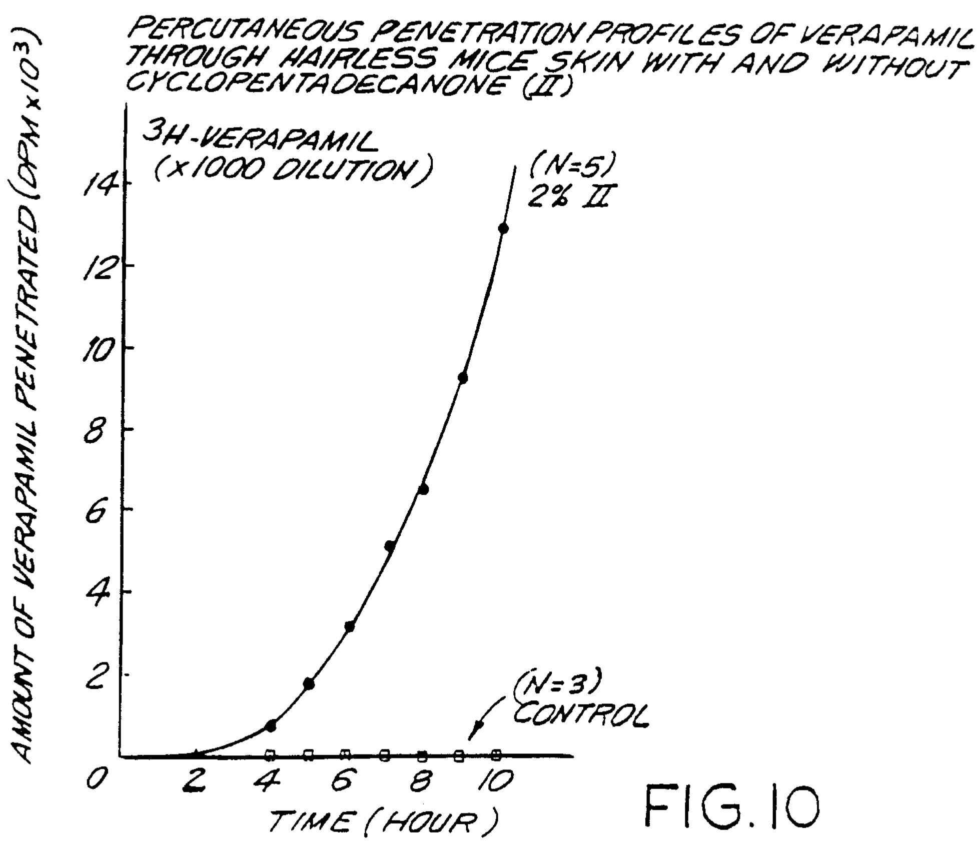
PERCUTANEOUS PENETRATION PROFILES OF VERAPAMIL THROUGH HAIRLESS MICE SKIN WITH AND WITHOUT CYCLOPENTADECANONE (II)
3H-VERAPAMIL
(x1000 DILUTION)
(N=5)
2% II
(N=3)
CONTROL
AMOUNT OF VERAPAMIL PENETRATED (DPM x10³)
TIME (HOUR)
0
2
4
6
8
10
12
14

FIG.10

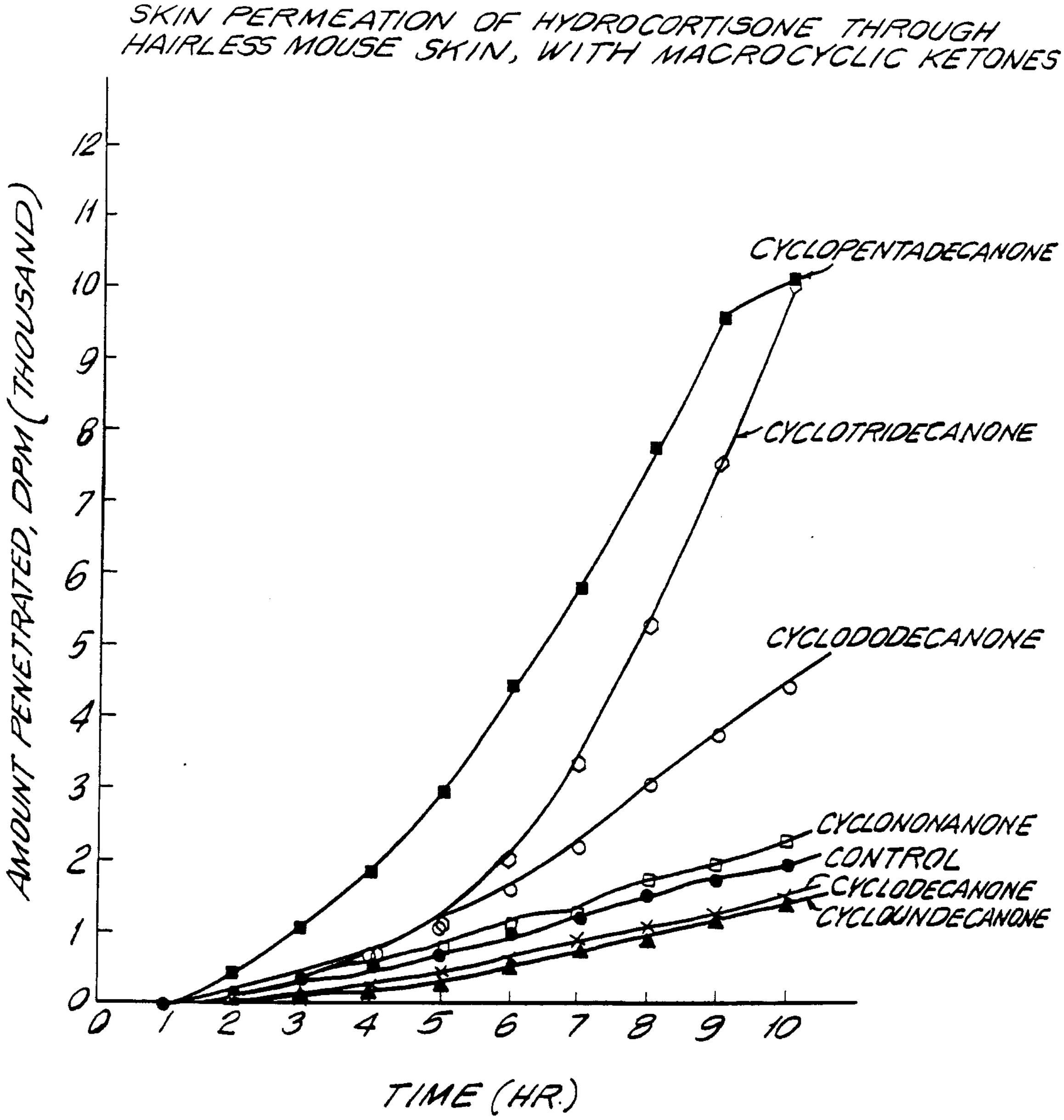
SKIN PERMEATION OF HYDROCORTISONE THROUGH HAIRLESS MOUSE SKIN, WITH MACROCYCLIC KETONES
AMOUNT PENETRATED, DPM (THOUSAND)
TIME (HR.)
0
1
2
3
4
5
6
7
8
9
10
11
12
CYCLOPENTADECANONE
CYCLOTRIDECANONE
CYCLODODECANONE
CYCLONONANONE
CONTROL
CYCLODECANONE
CYCLOUNDECANONE

FIG.11

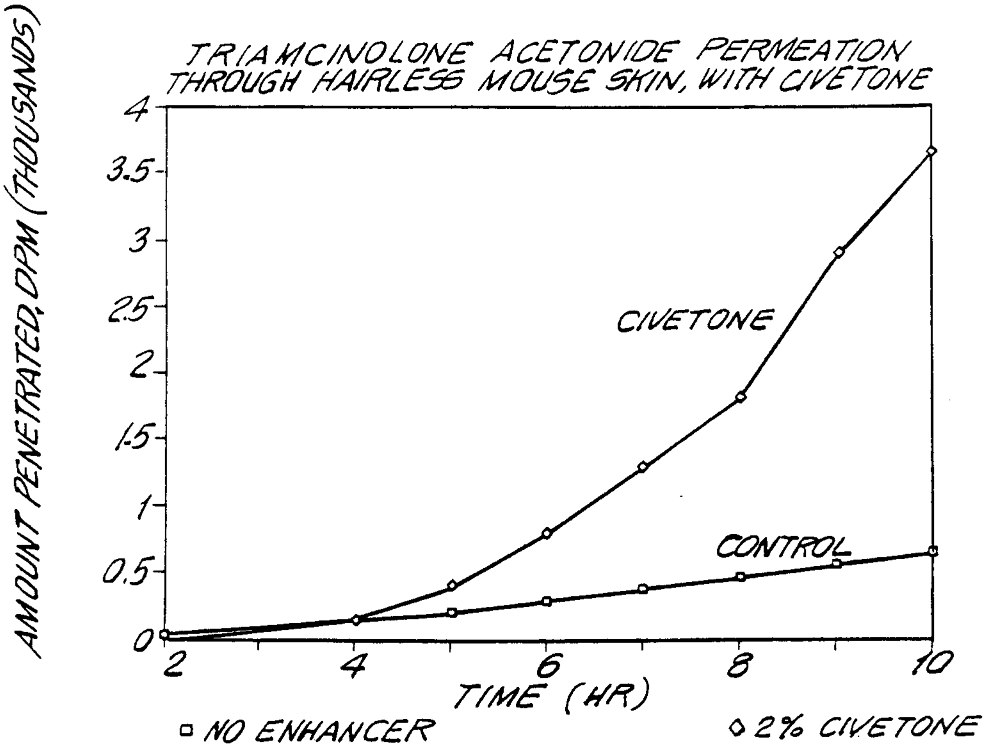
TRIAMCINOLONE ACETONIDE PERMEATION
THROUGH HAIRLESS MOUSE SKIN, WITH CIVETONE
AMOUNT PENETRATED, DPM (THOUSANDS)
4
3.5
3
2.5
2
1.5
1
0.5
0
2
4
6
8
10
TIME (HR)
CIVETONE
CONTROL
□ NO ENHANCER
◇ 2% CIVETONE

FIG.12

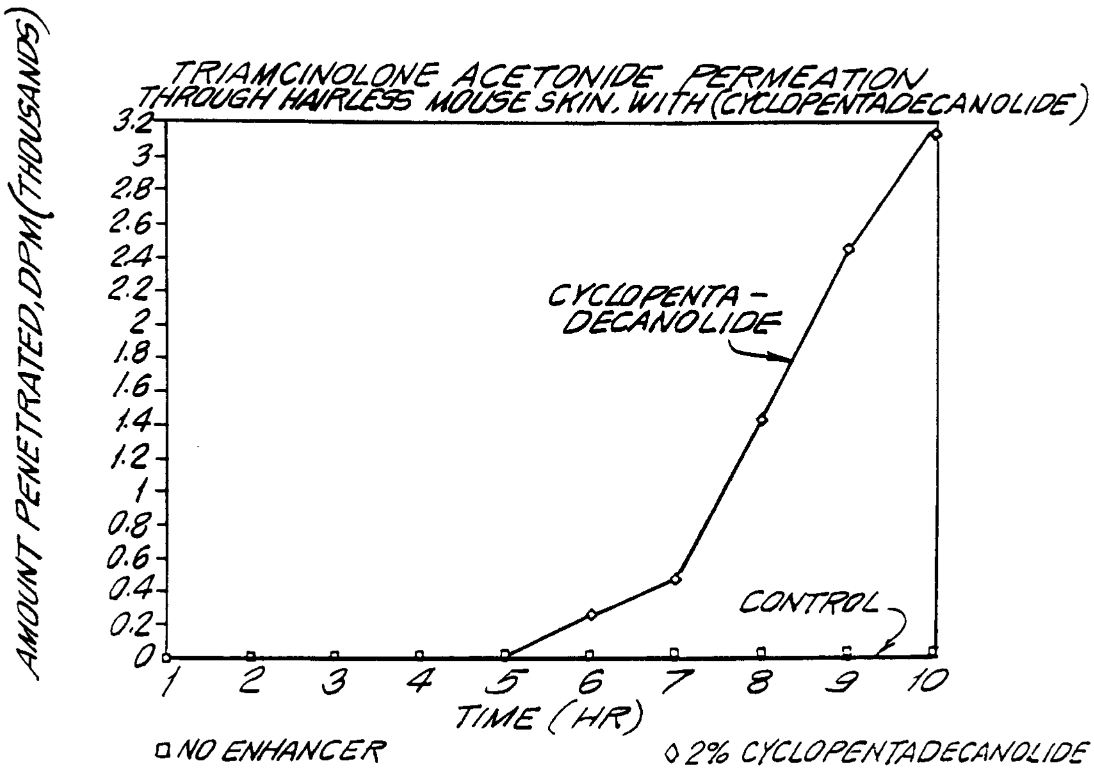
TRIAMCINOLONE ACETONIDE PERMEATION
THROUGH HAIRLESS MOUSE SKIN, WITH (CYCLOPENTADECANOLIDE)
AMOUNT PENETRATED, DPM (THOUSANDS)
3.2
3
2.8
2.6
2.4
2.2
2
1.8
1.6
1.4
1.2
1
0.8
0.6
0.4
0.2
0
1
2
3
4
5
6
7
8
9
10
TIME (HR)
CYCLOPENTA-
DECANOLIDE
CONTROL
□ NO ENHANCER
◇ 2% CYCLOPENTADECANOLIDE

FIG.13

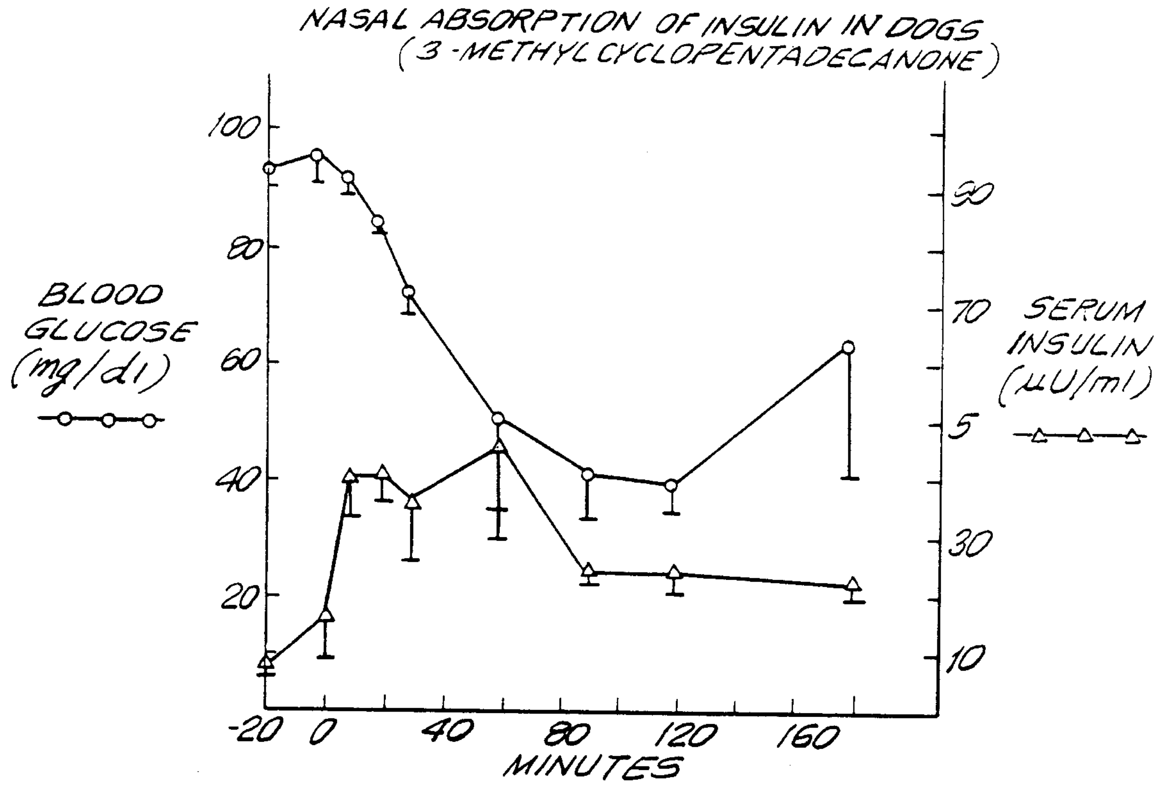
NASAL ABSORPTION OF INSULIN IN DOGS
(3-METHYLCYCLOPENTADECANONE)
BLOOD GLUCOSE (mg/dl)
SERUM INSULIN (µU/ml)
MINUTES
100
80
60
40
20
90
70
5
30
10
-20
0
40
80
120
160

FIG.14

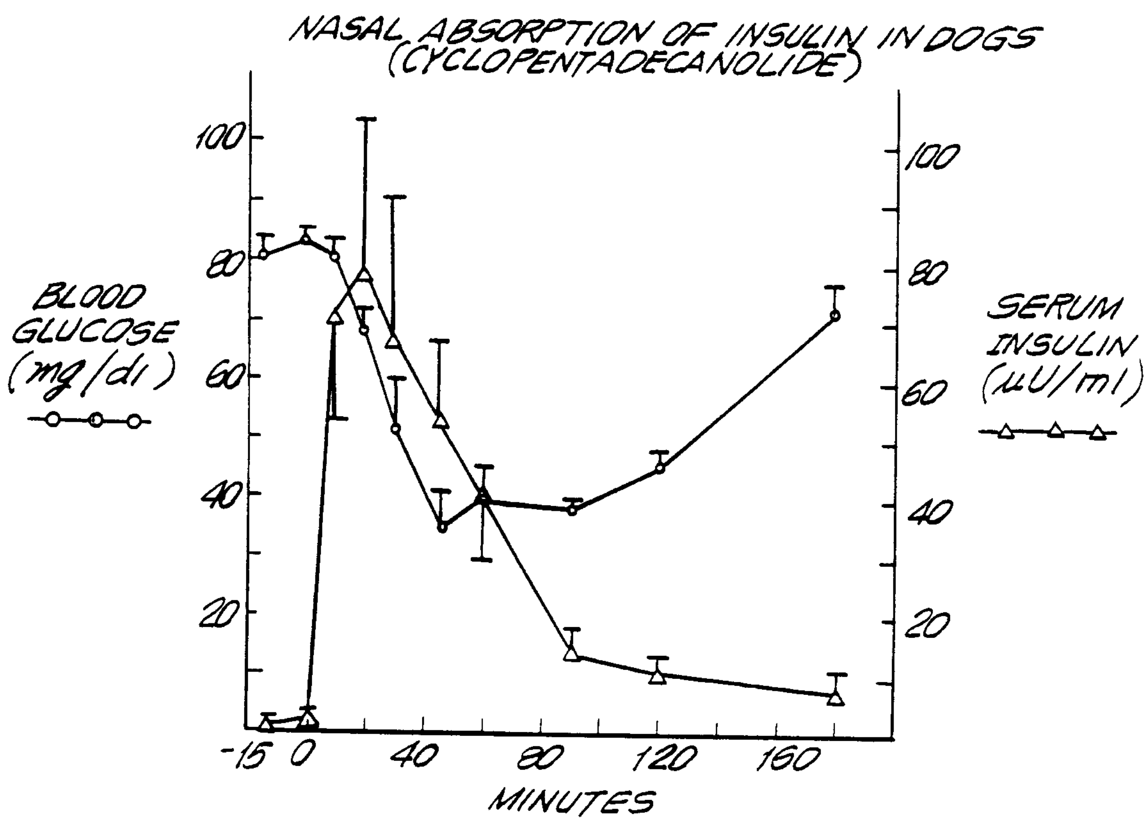
NASAL ABSORPTION OF INSULIN IN DOGS
(CYCLOPENTADECANOLIDE)
BLOOD GLUCOSE (mg/dl)
SERUM INSULIN (µU/ml)
MINUTES
100
80
60
40
20
-15
0
40
80
120
160

FIG.15